(19) Europäisches Patentamt
European Patent Office
Office européen des brevets

(11) EP 1 019 373 B1

# (12) FASCICULE DE BREVET EUROPEEN

(45) Date de publication et mention de la délivrance du brevet:
**12.11.2003 Bulletin 2003/46**

(21) Numéro de dépôt: **96931126.5**

(22) Date de dépôt: **13.09.1996**

(51) Int Cl.[7]: **C07D 211/32**, C07D 401/14, C07D 401/06, C07D 417/14, A61K 31/445, C07D 409/10, C07D 471/10, C07D 409/14, C07D 407/14, C07D 413/14

(86) Numéro de dépôt international:
**PCT/FR96/01416**

(87) Numéro de publication internationale:
**WO 97/010211 (20.03.1997 Gazette 1997/13)**

## (54) DERIVES DE 1-ACYL-3-PHENYL-3-(3-PIPERIDINOPROPYL)PIPERIDINE COMME ANTAGONISTES SELECTIFS DU RECEPTEUR NK3 HUMAIN

1-ACYL-3-PHENYL-3-(3-PIPERIDINOPROPYL)PIPERIDIN-DERIVATE ALS FÜR DEN MENSCHLICHEN NK3-REZEPTOR SELEKTIVE ANTAGONISTEN

1-ACYL-3-PHENYL-3-(3-PIPERIDINOPROPYL)PIPERIDINE DERIVATIVES AS HUMAN NK3 RECEPTOR SELECTIVE ANTAGONISTS

(84) Etats contractants désignés:
**AT BE CH DE DK ES FI FR GB GR IE IT LI LU MC NL PT SE**

(30) Priorité: **14.09.1995 FR 9510776**

(43) Date de publication de la demande:
**19.07.2000 Bulletin 2000/29**

(60) Demande divisionnaire:
**02010824.7 / 1 241 168**

(73) Titulaire: **SANOFI-SYNTHELABO**
**75013 Paris (FR)**

(72) Inventeurs:
- **BICHON, Daniel**
  **F-34000 Montpellier (FR)**
- **EDMONDS-ALT, Xavier**
  **F-34980 Combaillaux (FR)**
- **GUEULE, Patrick**
  **F-34820 Teyran (FR)**
- **PROIETTO, Vincenzo**
  **F-34680 Saint-Georges-d'Orques (FR)**
- **VAN BROECK, Didier**
  **F-34570 Murviel-les-Montpellier (FR)**

(74) Mandataire: **Gillard, Marie-Louise et al**
**Cabinet Beau de Loménie,**
**158, rue de l'Université**
**75007 Paris (FR)**

(56) Documents cités:

| | |
|---|---|
| **EP-A- 0 428 434** | **EP-A- 0 474 561** |
| **EP-A- 0 512 901** | **EP-A- 0 515 240** |
| **EP-A- 0 625 509** | **EP-A- 0 673 928** |
| **WO-A-93/18002** | **WO-A-94/26735** |

- **LIFE SCIENCES, vol. 56, no. 1, 1995, pages PL27-32, XP002005529 X. EMONDS-ALT ET AL.:**
- **CHEMICAL ABSTRACTS, vol. 123, no. 11, 11 Septembre 1995 Columbus, Ohio, US; abstract no. 132661j, XP002005530 & J. PHARMACOL. EXP. THER., vol. 274, no. 1, 1995, pages 148-154, F. OURY-DONAT ET AL.:**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des nouveaux composés antagonistes sélectifs du récepteur $NK_3$ humain pour la préparation de médicaments utiles dans le traitement de maladies psychiatriques, de maladies d'origine psychosomatique, de l'hypertension et d'une manière générale de toute pathologie centrale ou périphérique dans laquelle la neurokinine B et le récepteur $NK_3$ interviennent dans les régulations intemeuronales, un procédé pour leur obtention et les compositions pharmaceutiques en contenant en tant que principe actif.

Par maladie d'origine psychosomatique, on désigne, des maladies ayant leur origine dans le système nerveux central (SNC) et des conséquences pathologiques au niveau périphérique.

Dans les dernières années, de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces. Ainsi, les récepteurs $NK_3$ ont été identifiés chez le cobaye, le rat, le singe (Br. J. Pharmacol., 1990, 99, 767-773 ; Neurochem. Int., 1991, 18, 149-165) ; ils ont également été mis en évidence chez l'homme (FEBS Letters, 1992, 299(1), 90-95).

Une revue de C.A. Maggi et al. fait le point sur les récepteurs aux tachykinines et leurs antagonistes et expose les études pharmacologiques et les applications en thérapeutique humaine (J. Autonomie Pharmacol., 1993, 13, 23-93).

Parmi les antagonistes spécifiques du récepteur $NK_1$ on peut citer les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413).

Pour le récepteur $NK_2$, un antagoniste sélectif non peptidique, le SR 48968 a été décrit en détail (Life Sci., 1992, 50, PL101-PL106).

En ce qui concerne le récepteur $NK_3$ humain, un antagoniste sélectif non peptidique, le chlorhydrate de (+) -N-[1-[3-[1-benzoyl-3-(3,4-dichlorophényl) pipérid-3-yl]propyl]-4-phénylpipérid-4-yl]-N-méthylacétamide ou SR 142801 a été décrit (EP-A-0673 928 ; Peptides and their antagonists in tissue injury, Montreal, Canada, 1994, July 31-August 3. Canadian J. Physiol. Pharmacol., 1994, 72 (suppl. 2), 25, Abst. III. 0. 9. ; Life Sci., 1994, 56 (1), 27-32 ; British Pharmacol. Society, Canterbury, 1995, April 6-8 ; Eur. J. Pharmacol., 1995, 278 (1), 17-25 ; 1st. Eur. Congress Pharmacol., Milan, 1995, June 16-19).

Les demandes de brevet EP 474 561 et EP 512901 décrivent, des antagonistes des. neurokinines, plus particulièrement des antagonistes des récepteurs $NK_1$ ou $NK_2$. Les études pharmacologiques des antagonistes peptidiques et non-peptiques des récepteurs $NK_1$ et $NK_2$ ont montré que leurs affinités pour ces récepteurs ainsi que leurs activités pharmacologiques étaient très fortement fonction de l'espèce, très probablement suite à de faibles différences dans les séquences d'acides aminés, induisant ainsi de très fines variations structurales de ces récepteurs d'une espèce à l'autre (J. Autonomic Pharmacol., 1993, 13, 23-93). Certaines données expérimentales, confirmées par la caractérisation pharmacologique des composés objets de la présente invention, semblent indiquer qu'une situation comparable existe pour le récepteur $NK_3$. En particulier, le récepteur $NK_3$ humain se distingue du récepteur $NK_3$ du rat.

On a maintenant trouvé des composés non peptidiques qui présentent une très forte affinité pour le récepteur $NK_3$ humain et une grande spécificité pour ledit récepteur. Ces composés peuvent être utilisés pour la préparation de médicaments utiles dans le traitement de maladies psychiatriques ou d'origine psychosomatique et de toutes maladies centrales ou périphériques dans lesquelles la neurokinine B et le récepteur $NK_3$ interviennent dans les régulations intemeuronales.

Par très forte affinité pour le récepteur $NK_3$ humain on entend une affinité caractérisée par une constante d'inhibition Ki généralement inférieure à $5.10^{-9}$M.

Dans les études de fixation d'un ligand, la constante d'inhibition Ki est définie par la relation de Cheng-Prusoff (in Receptor Binding in Drug Research, eds. R.A. O'BRIEN. Marcel Dekker, New York, 1986):

$$Ki = \frac{IC_{50}}{1 + \frac{[L]}{Kd}}$$

[L] : concentration du ligand,
Kd : constante de dissociation du ligand,
$IC_{50}$ : concentration qui inhibe 50 % de la fixation du ligand.

Par grande spécificité pour le récepteur $NK_3$ humain, on entend que la constante d'inhibition (Ki) pour le récepteur $NK_3$ humain est généralement au moins 100 fois inférieure à la constante d'inhibition (Ki) pour le récepteur $NK_2$ ou à celle pour le récepteur $NK_1$ de différentes espèces.

Ainsi selon un de ses aspects, la présente invention a pour objet des composés de formule :

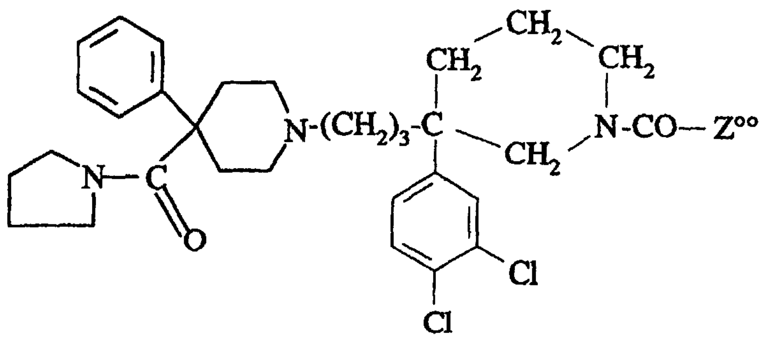

(I°°a)

dans laquelle :

- Z°° représente un 4-pyridyle, un 2-thiényle, un 3-thiényle, un 2-furyle ou un 3-furyle ;

ainsi que leurs sels éventuels avec des acides minéraux ou organiques, notamment les sels pharmaceutiquement acceptables.

Les composés de formule (I°°a) selon l'invention comprennent aussi bien les isomères optiquement purs que les racémiques.

On peut former des sels des composés de formule (I°°a). Ces sels comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels gharmaceutiquement acceptables, tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate, le glycolate, le gluconate, le citrate, l'iséthionate, le benzènesulfonate, le paratoluènesulfonate.

Les composés suivants :

la 3-(3,4-dichloroghényl)-1-isonicotinoyl-3-[3-[4-phényl-4-(pyrrolidin-1-ylcarbonyl)pipérid-1-yl]propyl]pipéridine ;
la 3-(3,4-dichlorophényl)-3-[3-[4-phényl-4-(pyrrolidin-1-ylcarbonyl)pipérid-1-yl] propyl]-1-(2-thénoyl)pipéridine ;
la 3-(3,4-dichlorophényl)-3-[3-[4-phényl-4-(pyrrolidin-1-ylcarbonyl)pipérid-1-yl]propyl]-1-(3-thénoyl)pipéridine ;
la 3-(3,4-dichlorophényl)-1-(2-furoyl)-3-[3-[4-phényl-4-(pyrrolidin-1-ylcarbonyl) pipérid-1-yl]propyl]pipéridine ;
la 3-(3,4-dichlorophényl)-1-(3-furoyl)-3-[3-[4-phényl-4-(pyrrolidin-1-ylcarbonyl) pipérid-1-yl]propyl]pipéridine ;

et leurs sels, notamment pharmaceutiquement acceptables sont tout particulièrement préférés, selon la présente invention.

L'invention concerne également, lorsqu'ils existent, les solvates des composés de formule (I°°a) de l'invention et de leurs sels.

Les composés selon l'invention sont obtenus par des méthodes connues en particulier celles qui sont décrites dans les demandes de brevet EP-A-474561 et EP-A-512901.

Un des procédés qui convient pour l'obtention des composés de formule (I°°a) et de leurs sels est décrit ci-après.

Selon ce procédé :

1) on traite un composé de formule :

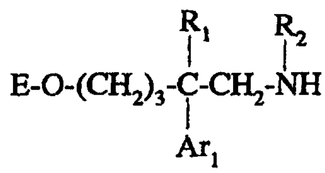 (II)

dans laquelle $Ar_1$ est le groupe 3,4-dichlorophényle; $R_1$ et $R_2$ représentent ensemble le groupe $-(CH_2)_3-$ et E représente l'hydrogène ou un groupe O-protecteur, avec un dérivé fonctionnel d'un acide de formule :

$$HO\text{-}CO\text{-}Z^{\circ\circ} \quad \text{(IIIa)}$$

dans laquelle Z°° est tel que défini précédemment,
pour obtenir un composé de formule :

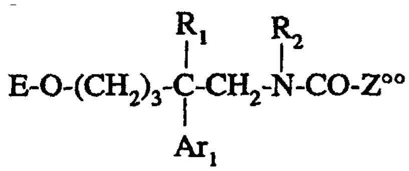 (IV)

2) on élimine éventuellement le groupe O-protecteur du composé de formule (IV), par action d'un acide ou d'une base, pour obtenir l'alcool de formule :

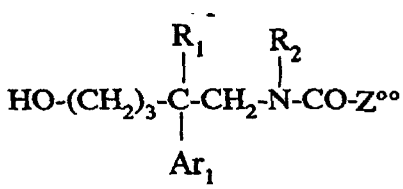 (V)

3) on traite l'alcool (V) avec un composé de formule :

$$G\text{-}SO_2\text{-}Cl \quad \text{(VI)}$$

dans laquelle G représente un groupe méthyle, phényle, tolyle ou trifluorométhyle, pour obtenir un composé de formule :

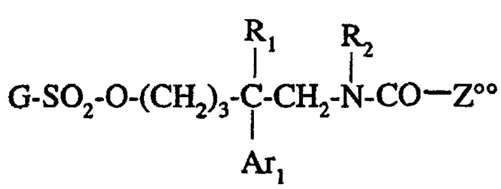 (VII)

4) on fait réagir le composé (VII)
avec une amine secondaire cyclique de formule :

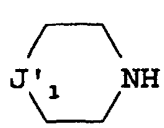 (VIIIa)

dans laquelle $J'_1$ représente un groupe :

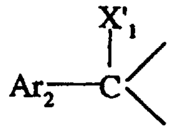

dans lequel $Ar_2$ est le groupe phényle et $X'_1$ est le groupe de formule $-CONR_{19}R_{20}$ dans lequel $R_{19}$ et $R_{20}$ ensemble avec l'atome d'azote auquel ils sont liés constituent l'hétérocycle pyrrolidine.
5) et on transforme éventuellement le produit ainsi obtenu en l'un de ses sels avec un acide minéral ou organique.

Selon une variante du procédé :

1') on protège l'atome d'azote du composé de formule (II) pour obtenir un composé de formule :

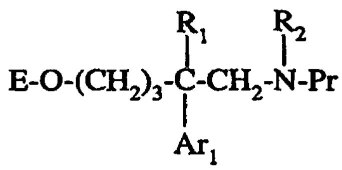 (XVII)

dans laquelle $Ar_1$ est le groupe 3,4-dichlorophényle, $R_1$ et $R_2$ forment ensemble le groupe $-(CH_2)_3-$ et E représente l'hydrogène ou un groupe O-protecteur, et Pr représente un groupe N-protecteur, tel que le groupe trityle, *tert*-butoxycarbonyle ou benzyloxycarbonyle,
2') on élimine éventuellement le groupe O-protecteur du composé de formule (XVII), par action d'un acide ou d'une base, pour obtenir l'acool de formule :

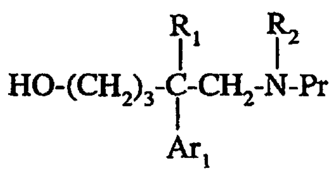 (XVIII)

3') on traite l'alcool (XVIII) avec un composé de formule (VI) tel que défini précédemment pour obtenir un composé de formule :

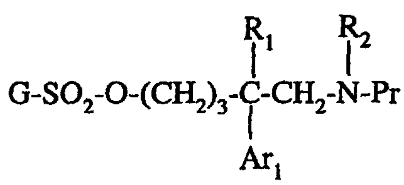 (XIX)

4') on fait réagir le composé (XIX) avec un composé de formule (VIIIa), tel que défini précédemment, pour obtenir un composé de formule :

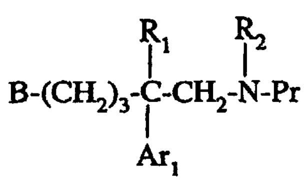 (XX)

dans laquelle $Ar_1$, $R_1$ et $R_2$ sont tels que définis précédemment et B est le groupe de formule :

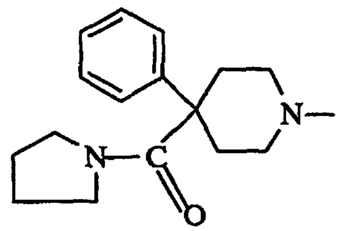

5') on élimine sélectivement le groupe protecteur Pr du composé de formule (XX), pour obtenir le composé de formule :

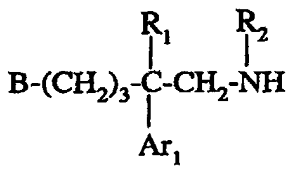

(XXI)

6') on traite le composé de formule (XXI) avec un composé (IIIa), tel que défini précédemment ;
7') et on transforme éventuellement le produit ainsi obtenu en l'un de ses sels avec un acide minéral ou organique.

Plus particulièrement, les composés de formule (I°°a) ainsi que leurs sels, notamment pharmaceutiquement acceptables sont préparés par la variante du procédé général décrite ci-dessus, selon laquelle :

1°) on fait réagir un composé de formule :

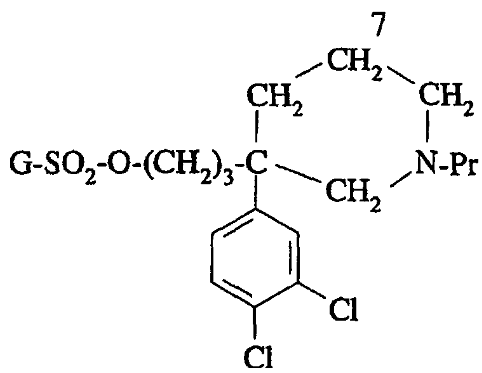

(XIX°)

dans laquelle G représente un groupe méthyle, phényle, tolyle ou trifluorométhyle et Pr représente un groupe N-protecteur, tel que le groupe trityle, *tert*-butoxycarbonyle ou benzyloxycarbonyle, avec un composé de formule :

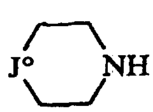

(VIII°)

dans laquelle J° a la même signification que $J'_1$ pour le composé (VIIIa), pour obtenir un composé de formule :

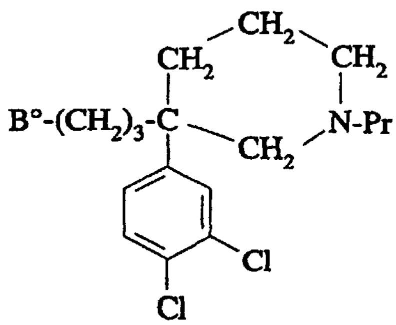

(XX°)

dans laquelle Pr est tel que défini précédemment et B° est le groupe de formule :

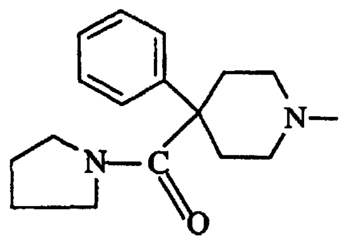

2°) on élimine sélectivement le groupe protecteur Pr du composé de formule (XX°), pour obtenir le composé de formule :

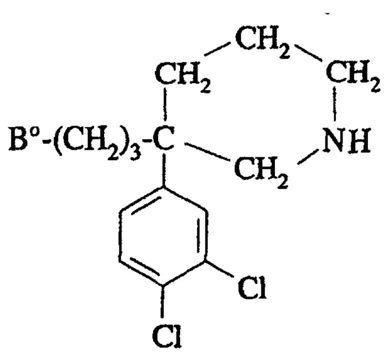

(XXI°)

3°) on traite le composé de formule (XXI°) avec un dérivé fonctionnel d'un acide de formule :

HO-CO-Z° (IIIa)

dans laquelle Z°° est tel que défini pour un composé de formule (I°°a);
4°) et on transforme éventuellement le produit (I°°a) ainsi obtenu en l'un de ses sels avec un acide minéral ou organique.

Au cours de l'une quelconque des étapes de préparation des composés de formule (I°°a) et plus particulièrement lorsqu'on met en oeuvre des composés intermédiaires de formule (II), (IV), (XX) ou (XX°) il peut être nécessaire et/ou souhaitable de protéger les groupes fonctionnels réactifs ou sensibles, tels que les groupes amine ou hydroxyle, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991. L'élimination des groupes protecteurs peut s'effectuer à une étape ultérieure opportune en utilisant les méthodes connues de l'homme de l'art et qui n'affectent pas le reste de la molécule concernée.
Ainsi, lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un ($C_1$-$C_4$) alkylcarbonyle.

Dans l'étape 1) ou dans l'étape 6') ou dans l'étape 3°) comme dérivé fonctionnel de l'acide (IIIa), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (IIIa) lui-même, on opère en présence d'un agent de couplage utilisé en chimie peptidique tel que le 1,3-dicyclohexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Dans l'étape 2) du procédé ou dans l'étape 2') de la variante, éventuellement on déprotège le composé de formule (IV) ou le composé de formule (XVII) ainsi obtenu selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe ($C_1$-$C_4$)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

Dans l'étape 3) du procédé ou dans l'étape 3') de la variante, la réaction de l'alcool de formule (V) ou de l'alcool de formule (XVIII) avec un chlorure de sulfonyle de formule (VI) s'effectue en présence d'une base telle que la triéthylamine, la pyridine, la N,N-diisopropyléthylamine ou la N-méthylmorpholine, dans un solvant inerte tel que le dichlorométhane, le benzène ou le toluène et à une température comprise entre -20°C et la température de reflux du solvant.

Dans l'étape 4) ou dans l'étape 4'), le composé (VII) ou le composé (XIX) ainsi obtenu est mis en réaction avec un composé de formule (VIIIa) ; dans l'étape 1°), le composé (XIX°) est mis en réaction avec un composé de formule (VIII°). La réaction s'effectue dans un solvant inerte tel que le N,N-diméthylformamide, l'acétonitrile, le chlorure de méthylène, le toluène, l'isopropanol ou un mélange de ces solvants et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (VIIIa), (VIII°) et éventuellement en présence d'un iodure de métal alcalin tel que l'iodure de potassium ou l'iodure de sodium. La réaction s'effectue à une température comprise entre la température ambiante et 100°C.

Dans l'étape 5') de la variante ou dans l'étape 2°), on déprotège le composé de formule (XX) obtenu ou le composé de formule (XX°) obtenu, selon les méthodes connues de l'homme de l'art.

On obtient finalement les composés de formule (I°°a) selon l'invention.

Les composés de formule (I°°a) sont isolés sous forme de base libre ou de sel selon les techniques classiques.

Ainsi lorsque le composé de formule (I°°a) est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane ou dans l'acétate d'éthyle, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2-sulfonate, le benzènesulfonate.

A la fin de la réaction, les composés de formule (I°°a) peuvent être isolés sous forme d'un de leurs sels, par exemple le chlorhydrate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) s'obtiennent par des méthodes connues en particulier celles qui sont décrites dans les demandes de brevet EP-A-0428434, EP-A-0474561, EP-A-0512901.

De façon particulière on peut préparer le composé de formule (II) dans laquelle E représente un hydrogène selon le SCHEMA 1 ci-après, dans lequel $Ar_1$ est le groupe 3,4-dichlorophényle.

SCHEMA 1

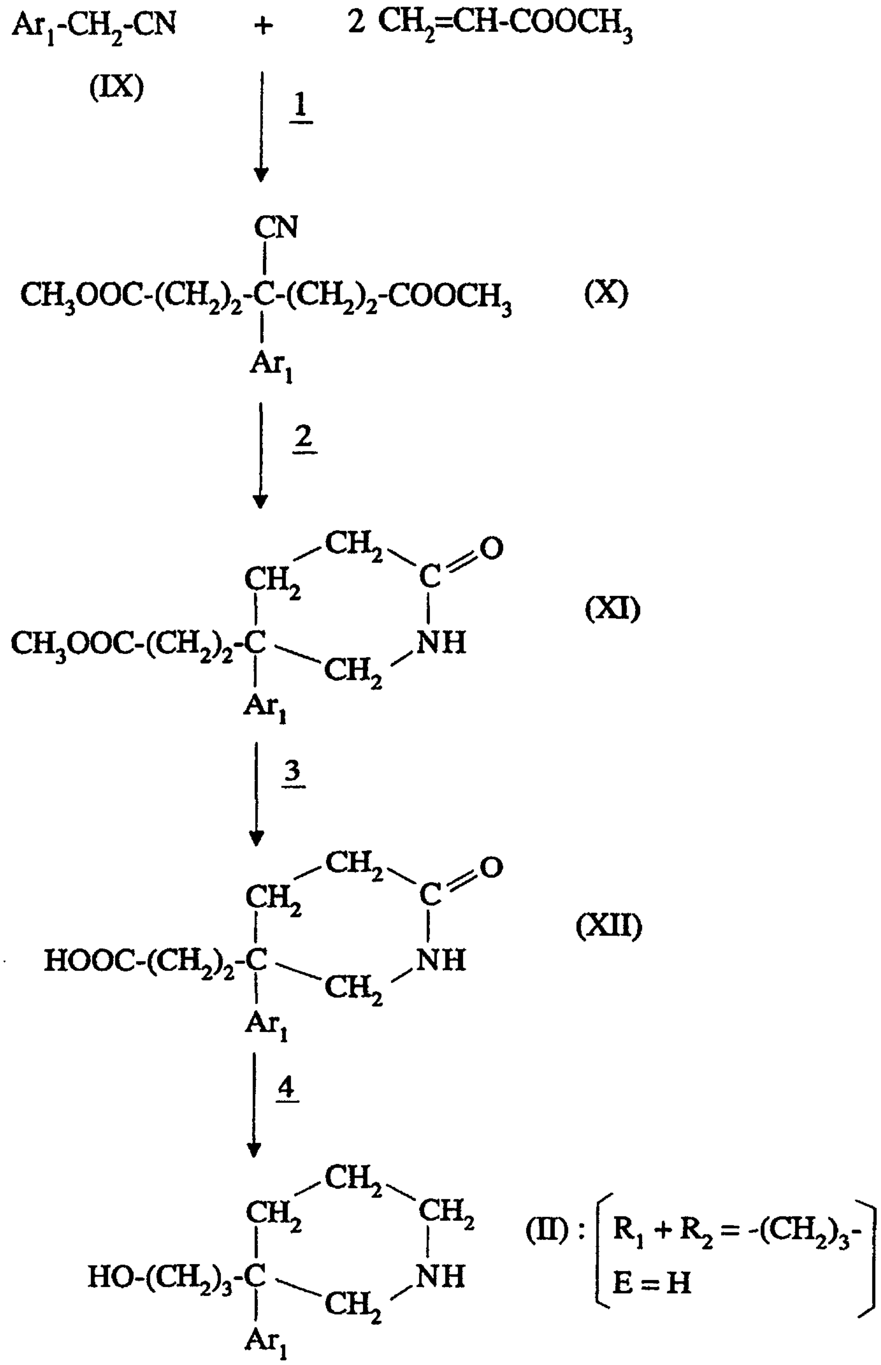


(IX)

(X)

(XI)

(XII)

(II) : $R_1 + R_2 = -(CH_2)_3-$ ; $E = H$

A l'étape 1, la réaction d'un composé de formule (IX) avec l'acrylate de méthyle en présence d'une base telle que le Triton®B ou le 1,8-diazabicyclo[5.4.0]undec-7-ène (DBU) permet d'obtenir le composé de formule (X). La réaction s'effectue dans un solvant inerte tel que le 1,4-dioxane ou le tétrahydrofurane et à une température comprise entre 60°C et la température de reflux du solvant.

A l'étape 2, le composé de formule (X) est soumis à une hydrogénation en présence d'un catalyseur tel que le nickel de Raney® pour obtenir le composé de formule (XI). La réaction s'effectue dans un solvant inerte tel qu'un alcanol, de préférence l'éthanol ou le 2-méthoxyéthanol, à une température comprise entre la température ambiante et 60°C et à une pression comprise entre la pression atmosphérique et 20 bars.

A l'étape 3, le composé de formule (XI) est soumis à une hydrolyse en milieu alcalin en utilisant par exemple une hydroxyde de métal alcalin tel que l'hydroxyde de sodium, ou l'hydroxyde de potassium dans un solvant tel que

l'eau, le méthanol ou un mélange de ces solvants et à une température comprise entre la température ambiante et la température de reflux du solvant.

Le composé de formule (XII) ainsi obtenu est réduit à l'étape 4 pour conduire au composé de formule (II) attendu. La réduction s'effectue au moyen d'un agent réducteur tel que l'hydrure d'aluminium et de lithium, l'hydrure de diisobutylaluminium, ou le borane dans le THF, dans un solvant inerte tel que le tétrahydrofurane, le 1,2-diméthoxyéthane ou le toluène à une température comprise entre la température ambiante et la température de reflux du solvant.

Les composés de formule (IIIa) sont connus ou préparés par des méthodes connues.

Les pipéridines de formule (VIIIa) sont connues ou préparées par des méthodes connues, telles que celles décrites dans EP-A-0428434, EP-A-0474561, EP-A-0512901 et EP-A-0515240.

On peut également préparer les pipéridines de formule (VIIIa) par des méthodes bien connues de l'homme de l'art, telles que celles décrites dans les publications suivantes :

J. Heterocyclic. Chem., 1986, 23, 73-75 ;
J. Chem. Soc., 1950, 1469;
J. Chem. Soc., 1945, 917 ;
J. Pharm. Sci., 1972, 61, 1316-1317 ;
J. Org. Chem., 1957, 22, 1484-1489 ;
Chem. Ber., 1975, 108, 3475-3482.

Pour préparer un composé de formule (VIIIa) dans laquelle X'$_1$ représente un groupe -CONR$_{19}$R$_{20}$, on fait réagir un composé de formule (VIIIa) dans laquelle X'$_1$ représente un carboxy avec un composé de formule HNR$_{19}$R$_{20}$ selon les méthodes bien connues de l'homme de l'art.

On peut préparer un composé de formule (VIIIa) dans laquelle X'$_1$ représente un carboxy par hydrolyse d'un composé de formule (VIIIa) dans laquelle X'$_1$ représente un cyano selon les méthodes connues de l'homme de l'art.

Les pipéridines de formule (VIII°) sont également connues ou peuvent être préparées selon des méthodes connues. Particulièrement, lorsque J° représente un groupe de structure :

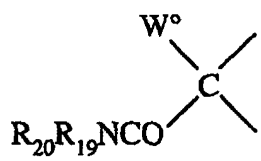

dans laquelle W° est le groupe phényle,
la pipéridine de formule (VIII°) est préparée selon une des méthodes décrites ci-dessus pour les composés de formule (VIIIa) dans laquelle X'$_1$ représente un groupe -CONR$_{19}$R$_{20}$, notamment par réaction d'un acide carboxylique de formule :

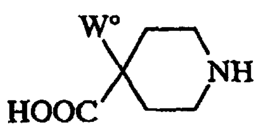

(VIIIa°)

avec un amine de formule NHR$_{19}$R$_{20}$.

Les énantiomères des composés selon l'invention, de formule :

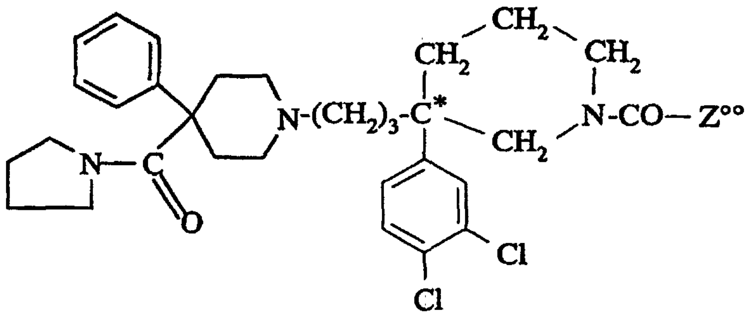

(I°°a*)

dans laquelle :

- "*" signifie que l'atome de carbone ainsi marqué a la configuration absolue (+) ou (-) déterminée ;
- Z°° est tel que défini pour les composés de formule (I°°a);

ainsi que leurs sels avec des acides minéraux ou organiques,
sont des composés nouveaux qui font partie de l'invention.

La résolution des mélanges racémiques des composés de formule (I°°a) permet d'isoler les énantiomères de formule (I°°a*). Il est cependant préférable d'effectuer le dédoublement des mélanges racémiques à partir d'un composé intermédiaire utile pour la préparation d'un composé de formule (I°°a) tel que décrit dans les demandes de brevet : EP-A-0474561, EP-A-0512901, EP-A-0591040 et EP-A-0612716.

Les composés de formule (I°°a) ci-dessus comprennent également ceux dans lesquels un ou plusieurs atomes d'hydrogène, de carbone ou d'iode ont été remplacés par leur isotope radioactif par exemple le tritium, le carbone-14 ou l'iode-125. De tels composés marqués sont utiles dans des travaux de recherche, de métabolisme ou de pharmacocinétique, dans des essais biochimiques en tant que ligands de récepteurs.

L'affinité des composés de formule (I°°a) pour les récepteurs aux tachykinines a été évaluée in vitro par plusieurs essais biochimiques utilisant des radioligands :

1°) La liaison de [$^{125}$I] BH-SP (Substance P marquée à l'iode 125 à l'aide du réactif de Bolton-Hunter) aux récepteurs $NK_1$ du cortex de rat, de l'iléon de cobaye et des cellules lymphoblastiques humaines.
2°) La liaison de [$^{125}$I] His-$NK_A$ aux récepteurs $NK_2$ de vessie de rat ou la liaison de [$^{125}$I]NPγ aux récepteurs $NK_2$ de l'iléon de cobaye.
3°) La liaison de [$^{125}$I] His [MePhe$^7$] $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol,. 1993, 250, 403-413).

Les composés selon l'invention inhibent fortement la liaison de [$^{125}$I]His[MePhe$^7$] $NK_B$ aux récepteurs $NK_3$ du cortex cérébral de cobaye et de gerbille ainsi qu'aux récepteurs clonés $NK_3$ humains : la constante d'inhibition Ki est généralement inférieure à $5.10^{-9}$M. Pour les mêmes composés on a constaté que la constante d'inhibition (Ki) pour les récepteurs $NK_3$ du cortex cérébral de rat est généralement supérieure à $10^{-8}$M et que la constante d'inhibition (Ki) pour le récepteur $NK_2$ du duodénum de rat et les récepteurs $NK_1$ du cortex de rat est généralement supérieure ou égale à $10^{-7}$ M.

Les composés selon la présente invention ont également été évalués in vivo sur deux modèles animaux.

Chez le gerbille, un comportement de rotation est induit par administration intrastriatale d'un agoniste spécifique du récepteur $NK_3$ : le senktide ; on a constaté qu'une application unilatérale de senktide dans le striatum de gerbille conduit à de fortes rotations contralatérales qui sont inhibées par les composés selon l'invention administrés soit par voie intrapéritonéale, soit par voie orale.

Ce résultat montre que les composés selon l'invention passent la barrière hématoméningée et qu'ils sont susceptibles de bloquer, au niveau du système nerveux central, l'action propre aux récepteurs $NK_3$. Ils pourront ainsi être utilisés pour le traitement de toute pathologie centrale $NK_B$ dépendante, telle que les maladies psychiatriques, ou de toute pathologie médiée au niveau central par le récepteur $NK_3$, telle que les maladies psychosomatiques.

Chez le cobaye, une injection de senktide par voie intraveineuse ou intracérébroventriculaire induit une hypertension qui est supprimée par l'administration par voie orale ou intraveineuse des composés selon l'invention.

Ce résultat montre que les composés selon l'invention agissent au niveau cardiovasculaire et qu'ils sont capables de bloquer l'action propre aux récepteurs $NK_3$ à ce niveau, notamment l'hypertension (Nakayama et al., Brain

Res. 1992, 595 339-342, Takano and Kamiya, Asia Pacific. J. Pharmacol., 1991, 6, 341-346, Saigo et al., Neuroscience Letters, 1993, 159, 187-190).

Chez le cobaye, une inhalation, par exemple, de Substance P induit une hyperactivité bronchique à l'acétylcholine ainsi qu'une hypersensibilité à l'histamine par exemple de l'extravasation plasmatique. Un antagoniste $NK_3$ bloque ces deux processus caractéristiques des pathologies respiratoires comme l'asthme.

Dans ces tests, les composés selon l'invention sont actifs à des doses variant de 0,1 mg à 30 mg par kg par voie orale, intraveineuse ou intrapéritonéale.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Selon un autre de ses aspects, la présente invention concerne des compositions pharmaceutiques renfermant, en tant que principe actif, un composé de formule (I) ou un de ses sels pharmaceutiquement acceptables, ayant une affinité très forte pour le récepteur $NK_3$ humain, caractérisée par une constante d'inhibition Ki généralement inférieure à $5.10^{-9}$M dans les études de fixation du ligand.

Les composés de formule (I°°a) et leurs sels pharmaceutiquement acceptables peuvent être utilisés à des doses journalières de 0,01 à 100 mg par kilo de poids corporel du mammifère à traiter, préférentiellement à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Les maladies pour le traitement desquelles les composés et leurs sels pharmaceutiquement acceptables peuvent être utilisés, sont par exemple les maladies associées à un dysfonctionnement des systèmes dopaminergiques telle que la schizophrénie, la maladie de Parkinson, les maladies associées à un dysfonctionnement des systèmes noradrénergiques et sérotoninergiques tels que l'anxiété, les troubles de la vigilance, de l'humeur, ainsi que les maladies épileptiques de toute forme et en particulier le Grand Mal, la démence, les maladies neurodégénératives, et les maladies périphériques dans lesquelles la participation du système nerveux central et/ou du système nerveux périphérique se fait par l'intermédiaire de la neurokinine B agissant comme neurotransmetteur ou neuromodulateur tels que la douleur, la migraine, l'inflammation aigue ou chronique, les troubles cardiovasculaires en particulier l'hypertension, l'insuffisance cardiaque, et les troubles du rythme, les troubles respiratoires (asthme, rhinite, toux, bronchites, allergies, hypersensibilité), les troubles du système gastrointestinal tels que ulcère oesophagique, colite, désordres liés au stress (stress-related disorders), syndrome du colon irritable (IBS), hypersécrétion acide (acidic secretion), emesis/nausée (consécutive à la chimiothérapie ou post opératoire, due au mal du transport ou aux troubles vestibulaires), les troubles du système urinaire (incontinence, vessie neurologique), les maladies du système immunitaire (arthrite rhumatoide), et plus généralement toute pathologie neurokinine B dépendante.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycerol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions injectables qui contiennent des agents de dispersion et/ou des agents mouillants pharmacologiquement compatibles, par exemple le propylèneglycol ou le butylèneglycol.

Pour une administration par inhalation on utilise un aérosol contenant en outre, par exemple du trioléate de

sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif, seul ou associé à un excipient, sous forme de poudre.

Le principe actif peut être également présenté sous forme de complexe avec une cyclodextrine, par exemple, α, β, γ,-cyclodextrine, 2-hydroxypropyl-β-cyclodextrine, méthyl-β-cyclodextrine.

Le principe actif peut être formulé également sous forme de microcapsules, éventuellement avec un ou plusieurs supports ou additifs.

Dans chaque unité de dosage le principe actif de formule (I°°a) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions susdites peuvent également renfermer d'autres produits actifs utiles pour la thérapeutique souhaitée tels que, par exemple, des bronchodilatateurs, des antitussifs ou des antihistaminiques.

Grâce à leur très forte affinité pour le récepteur $NK_3$ humain et à leur grande sélectivité, les composés selon l'invention pourront être utilisés, sous forme radiomarquée comme réactifs de laboratoire.

Par exemple, ils permettent d'effectuer la caractérisation, l'identification et la localisation du récepteur $NK_3$ humain dans des coupes de tissus, ou du récepteur $NK_3$ chez l'animal entier par autoradiographie.

Les composés selon l'invention permettent également d'effectuer le tri ou screening des molécules en fonction de leur affinité pour le récepteur $NK_3$ humain. On opère alors par une réaction de déplacement du ligand radiomarqué, objet de la présente invention de son récepteur $NK_3$ humain.

Dans les Préparations et dans les exemples on utilise les abréviations suivantes :

EtOH : éthanol
MeOH : méthanol
Ether : éther diéthylique
Ether iso : éther diisopropylique
DMF : diméthylformamide
DCM : dichlorométhane
THF : tétrahydrofurane
AcOEt : acétate d'éthyle
$K_2CO_3$ : carbonate de potassium
NaCl : chlorure de sodium
$Na_2SO_4$ : sulfate de sodium
$MgSO_4$ : sulfate de magnésium
NaOH : soude
HCl : acide chlorhydrique
BOP : benzotriazol-1-yloxytris(diméthylamino)phosphonium hexafluorophosphate
DBU : 1,8-diazabicyclo[5.4.0]undec-7-ène
F : point de fusion
TA : température ambiante

PREPARATION 1.1

1-Benzyl-méthylamino)-4-phénylpipéridine.

A) 1-Benzyl-4-hydroxy-4-phénylpipéridine.

Ce composé est préparé par action du phényllithium sur la 1-benzylpipérid-4-one selon le procédé décrit dans EP-A-474561.

B) 4-Acétamido-1-benzyl-4-phénylpipéridine.

Ce composé est préparé par action de l'acétonitrile sur le composé obtenu à l'étape précédente selon le procédé décrit dans EP-A-474561.

C) Dichlorhydrate de 4-amino-1-benzyl-4-phénylpipéridine.

On chauffe à reflux pendant 48 heures un mélange de 50 g du composé obtenu à l'étape précédente, 90 ml d'une solution d'HCl concentrée dans 210 ml d'eau. On concentre sous vide le mélange réactionnel, reprend le résidu dans un mélange EtOH/toluène et évapore sous vide les solvants. On dissout le résidu dans 100 ml de MeOH chaud, ajoute 500 ml d'acétone et laisse sous agitation en refroidissant au bain de glace. On essore les cristaux formés, les lave à l'acétone puis à l'éther et sèche. On obtient 48,9 g du produit attendu.

D) 1-Benzyl-4-(formylamino)-4-phénylpipéridine.

A une solution de 48,9 g du composé obtenu à l'étape précédente et 25 g de formiate de sodium dans 340 ml d'acide formique, on ajoute goutte à goutte 110 ml d'anhydride acétique puis laisse une nuit sous agitation à TA. On concentre sous vide le mélange réactionnel, reprend le résidu à l'eau, alcalinise par ajout d'une solution de NaOH concentrée, extrait au DCM, sèche la phase organique sur $MgSO_4$ et évapore sous vide le solvant. On obtient 38,8 g du produit attendu après cristallisation dans le mélange éther iso/pentane, F = 140°C.

E) 1-Benzyl-4-(méthylamino)-4-phénylpipéridine.

A une suspension de 12,5 g d'hydrure d'aluminium et de lithium dans 100 ml de THF, on ajoute lentement une solution de 38,8 g du composé obtenu à l'étape précédente dans 400 ml de THF et chauffe à reflux pendant 3 heures. Après refroidissement, on ajoute au mélange réactionnel une solution de 5 ml de NaOH concentrée dans 45 ml d'eau, filtre les sels minéraux et concentre sous vide le filtrat. On obtient 38 g du produit attendu.

PREPARATION 1.2

*p*-Toluènesulfonate de 4-(acétyl-N-méthylamino)-4-phénylpipéridine.

A) 4-(Acétyl-N-méthylamino)-1-benzyl-4-phénylpipéridine.

On refroidit à 0-5°C une solution de 30 g du composé obtenu à l'étape E de la PREPARATION 1.1 et 16,5 ml de triéthylamine dans 300 ml de DCM, ajoute goutte à goutte 8 ml de chlorure d'acétyle et laisse 30 minutes sous agitation à TA. On lave deux fois le mélange réactionnel à l'eau, par une solution de NaOH 2N, sèche la phase organique sur $MgSO_4$ et évapore sous vide le solvant. On obtient 31,6 g du produit attendu après cristallisation dans le mélange éther iso/pentane, F = 104°C.

B) *p*-Toluènesulfonate de 4-(acétyl-N-méthylamino)-4-phénylpipéridine.

On hydrogène pendant 3 heures à 25°C et à pression atmosphérique un mélange de 5 g du composé obtenu à l'étape précédente, 2,9 g d'acide *p*-toluènesulfonique monohydrate, 0,5 g de palladium sur charbon à 10 % et 80 ml d'EtOH. On filtre le catalyseur et concentre sous vide le filtrat. On obtient 5,7 g du produit attendu après cristallisation dans l'acétone, F = 165°C.

PREPARATION 1.3

4-Phényl-4-(pyrrolidin-1-ylcarbonyl)pipéridine, hémihydrate.

A) 1-*tert*-Butoxycarbonyl-4-carboxy-4-phénylpipéridine.

A un mélange de 30 g de *p*-toluènesulfonate de 4-carboxy-4-phénylpipéridine dans 300 ml de dioxane, on ajoute 30 ml d'eau, 32,9 g de $K_2CO_3$, puis chauffe à 60°C et ajoute goutte à goutte 18,2 g de di-*tert*-butyldicarbonate. On chauffe ensuite 2 heures à 60°C puis 30 minutes à reflux. Après refroidissement à TA, on concentre sous vide le mélange réactionnel, extrait le résidu au DCM, lave la phase organique par une solution tampon pH = 2, acidifie à pH = 4 par ajout d'HCl 2N, lave par une solution tampon pH = 2, à l'eau, par une solution saturée de NaCl, sèche sur $MgSO_4$ et évapore sous vide le solvant. On obtient 23,7 g du produit attendu.

B) 1-*tert*-Butoxycarbonyl-4-(pyrrolidin-1-ylcarbonyl)-4-phénylpipéridine.

A une solution de 14 g du composé obtenu à l'étape précédente dans 200 ml de DCM, on ajoute 9,29 g de

triéthylamine puis 3,27 g de pyrrolidine. On refroidit au bain de glace, ajoute 22,4 g de BOP et laisse sous agitation en laissant remonter la température à TA. On concentre sous vide le mélange réactionnel, extrait le résidu au DCM, lave la phase organique à l'eau, trois fois par une solution de NaOH à 10 %, à l'eau, trois fois par une solution tampon pH = 2, à l'eau, par une solution saturée de NaCl, sèche sur $MgSO_4$ et évapore sous vide le solvant. On obtient 16,4 g du produit attendu.

C) 4-Phényl-4-(pyrrolidin-1-ylcarbonyl)pipéridine, hémihydrate.

A une solution de 16,4 g du composé obtenu à l'étape précédente dans 200 ml de MeOH, on ajoute jusqu'à pH = 1 une solution d'HCl concentrée et laisse 5 heures sous agitation à TA. On concentre sous vide le mélange réactionnel, reprend le résidu à l'acétone et évapore sous vide le solvant. On obtient un solide blanc que l'on recristallise dans le propan-2-ol. On reprend le produit obtenu par une solution de NaOH à 10 %, extrait au DCM, lave la phase organique par une solution de NaOH à 10 %, par une solution saturée de NaCl, sèche sur $MgSO_4$ et évapore sous vide le solvant. On obtient 7 g du produit attendu après cristallisation dans l'éther, F = 126°C.

PRÉPARATION 2

Chlorhydrate de 3-(3,4-dichlorophényl)-3-(3-hydroxypropyl)pipéridine.

A) 4-Cyano-4-(3,4-dichlorophényl)heptanedioate de méthyle.

Dans un tricol, on dissout 37,2 g de 3,4-dichlorophénylacétonitrile et 34,43 g d'acrylate de méthyle dans 20 ml de dioxane ; on ajoute 1 ml de DBU, chauffe 2 heures à 60°C, évapore, dilue avec 400 ml d'acétate d'éthyle puis lave avec HCl dilué, une solution de NaCl, sèche sur $MgSO_4$ et évapore. Le produit attendu est cristallisé dans 100 ml d'acétate d'éthyle, et 100 ml d'éther avec 100 ml d'heptane. On obtient 47 g du produit.

B) 3-[5-(3,4-dichlorophényl)-2-oxopipérid-5-yl]propionate de méthyle.

On dissout 40 g du composé préparé à l'étape A dans 500 ml de 2-méthoxyéthanol, on ajoute 2 g de Nickel de Raney® et hydrogène à 40°C sous pression atmosphérique pendant 3 jours. On filtre, évapore, et obtient le produit attendu sous forme d'huile (39 g).

C) Acide 3-[5-(3,4-dichlorophényl)-2-oxopipérid-5-yl]propanoïque.

On dissout 17 g du composé préparé à l'étape précédente dans 250 ml de méthanol, ajoute 2,8 g de potasse et 10 ml d'eau puis on porte à reflux pendant 2 heures. On évapore à sec, reprend l'huile obtenue par 200 ml d'eau et lave par 100 ml d'acétate d'éthyle. La phase aqueuse est acidifiée par une solution d'HCl à 30 % puis on filtre et sèche le précipité formé. On recristallise dans le méthanol à chaud et obtient 18,3 g du composé attendu.

D) Chlorhydrate de 3-(3,4-dichlorophényl)-3-(3-hydroxypropyl)pipéridine.

On dissout 5 g du composé obtenu à l'étape précédente dans 20 ml de THF, ajoute 75 ml de borane (concentration 1 M dans le THF) et chauffe au reflux pendant 24 heures, sous azote. On ajoute 25 ml de méthanol, 50 ml d'HCl 4N et laisse sous agitation 30 minutes puis on ajoute de la soude à 40 % jusqu'à un pH supérieur à 10. On extrait 3 fois par 150 ml de DCM, sèche la phase organique sur $MgSO_4$ et évapore. Le résidu est mis en solution dans DCM avec une solution 4N d'HCl dans l'éther. Après évaporation, on obtient une mousse et le produit attendu (4,5 g) cristallise dans le mélange AcOEt/éther.

EXEMPLE 1

Dichlorhydrate de 3-(3,4-dichlorophényl)-1-isonicotinoyl-3-[3-[4-phényl-4-(pyrrolidin-1-ylcarbonyl)pipérid-1-yl]propyl] pipéridine, trihydrate, isomère (+) (composé de formule I°°a, Z°° = 4-pyridyle).

A) Chlorhydrate de 3-(3,4-dichlorophényl)-3-(3-hydroxypropyl)pipéridine, isomère (+).

A une solution de 10 g du composé obtenu à la PREPARATION 2 dans 20 ml d'eau, on ajoute 5 ml d'une solution à 40 % de NaOH, extrait au DCM, sèche la phase organique sur $MgSO_4$, évapore sous vide le solvant et obtient 9 g d'huile. On dissout 2,7 g de l'huile ainsi obtenue dans 50 ml de propan-2-ol, ajoute 2,36 g d'acide 10-camphosulfonique isomère (+) et chauffe à reflux. Après refroidissement, cristallisation et essorage des cristaux formés (3,86 g), on dissout ces derniers dans une solution à 10 % de NaOH, extrait au chloroforme, sèche la phase organique sur $MgSO_4$ et évapore sous vide le solvant. On obtient 2,3 g du produit sous forme d'huile dont on fait le chlorhydrate. On mesure le pouvoir rotatoire du chlorhydrate.

$\alpha_D^{25}$ = + 5,5° (c = 0.1 ; MeOH)

Une deuxième cristallisation est effectuée à partir de 2,12 g de l'huile obtenue et 1,84 g d'acide 10-camphosulfonique isomère (+) dans 40 ml de propan-2-ol. Après alcalinisation par NaOH, extraction au chloroforme, séchage sur $MgSO_4$ et évaporation, on obtient 2,1 g du produit attendu sous forme d'huile dont on fait le chlorhydrate.

$\alpha_D^{25}$ = + 6,5° (c = 0,1 ; MeOH)

B) 1-(*tert*-Butoxycarbonyl)-3-(3,4-dichlorophényl)-3-(3-hydroxypropyl)pipéridine, isomère (+).

A une solution de 0,9 g du composé obtenu à l'étape précédente et 0,6 g de triéthylamine dans 100 ml de DCM, on ajoute 0,61 g de di-*tert*-butyl dicarbonate et laisse 30 minutes sous agitation à TA. On concentre sous vide, extrait le résidu à l'AcOEt, lave la phase organique par une solution tampon pH = 2, par une solution de NaOH 1N, par une solution saturée de NaCl, sèche sur $MgSO_4$ et évapore sous vide le solvant. On obtient 1,1 g du produit attendu sous forme d'huile.

C) 1-(*tert*-Butoxycarbonyl)-3-(3,4-dichlorophényl)-3-[3-(méthanesulfonyloxy)propyl] pipéridine, isomère (+).

On refroidit à 0-5°C une solution de 22 g du composé obtenu à l'étape précédente et 6,86 g de triéthylamine dans 150 ml de DCM, ajoute 7,13 g de chlorure de méthanesulfonyle et laisse 1 heure sous agitation à 0-5°C puis 2 heures à TA. On concentre sous vide, extrait le résidu à l'AcOEt, lave la phase organique à l'eau, par une solution saturée de NaCl, sèche sur $MgSO_4$ et évapore sous vide le solvant. On reprend le produit obtenu à l'éther puis évapore sous vide. On obtient 26,4 g du produit attendu sous forme d'huile.

D) 1-(*tert*-Butoxycarbonyl)-3-(3,4-dichlorophényl)-3-[3-[4-phényl-4-(pyrrolidin-1-ylcarbonyl)pipérid-1-yl]propyl]pipéridine, isomère (+).

On chauffe à 100°C pendant 3 heures et 30 minutes un mélange de 17 g de 4-phényl-4-(pyrrolidin-1-ylcarbonyl)pipéridine, 25,5 g du composé obtenu à l'étape précédente et 22,7 g de $K_2CO_3$ dans 100 ml du mélange DMF/acétonitrile (50/50 ; v/v). On concentre sous vide, extrait le résidu à l'AcOEt, lave la phase organique à l'eau, par une solution saturée de NaCl, sèche sur $MgSO_4$ et évapore sous vide le solvant. On reprend le résidu au pentane et essore le précipité formé. On obtient 33 g du produit attendu, F = 133-137°C.

$\alpha_D^{20}$ = + 33,2° (c = 0,5 ; MeOH)

E) Dichlorhydrate de 3-(3,4-dichlorophényl)-3-[3-[4-phényl-4-(pyrrolidin-1-ylcarbonyl) pipérid-1-yl]propyl]pipéridine, isomère (+).

A une solution de 25,8 g du composé obtenu à l'étape précédente dans 200 ml de MeOH, on ajoute jusqu'à pH = 1 une solution concentrée d'HCl, et chauffe à 40°C pendant 3 heures. On concentre sous vide et cristallise le produit obtenu dans un mélange AcOEt/éther. On obtient 17 g du produit attendu, F = 170°C.

$\alpha_D^{20}$ = + 13° (c = 0,5 ; MeOH)

F) Dichlorhydrate de 3-(3,4-dichlorophényl)-1-isonicotinoyl-3-[3-[4-phényl-4-(pyrrolidin-1-ylcarbonyl)pipérid-1-yl]propyl]pipéridine, trihydrate, isomère (+).

On refroidit à 0-5°C une solution de 1,7 g du composé obtenu à l'étape précédente et 0,9 g de triéthylamine dans 10 ml de DCM, ajoute 0,6 g de chlorure chlorhydrate d'isonicotinoyle et laisse 1 heure sous agitation à TA. On concentre sous vide, extrait le résidu à l'AcOEt, lave la phase organique à l'eau, par une solution saturée de NaCl, sèche sur $Na_2SO_4$ et évapore sous vide le solvant. On chromatographie le résidu sur silice H en éluant au DCM puis par le mélange DCM/MeOH (93/7 ; v/v). On dissout le produit obtenu dans l'AcOEt, fait barbotter un courant d'HCl gaz jusqu'à pH = 1 et ajoute de l'éther jusqu'à précipitation. On obtient 0,27 g du produit attendu après essorage et séchage, F = 140-142°C.

$\alpha_D^{20}$ = + 11,6° (c = 0,5 ; MeOH)

En procédant selon le mode opératoire décrit à l'étape F de l'EXEMPLE 1, à partir du composé obtenu à l'étape E de l'EXEMPLE 1 et des chlorures d'acides appropriés, on prépare les composés selon l'invention rassemblés dans le TABLEAU I ci-après.

## TABLEAU I

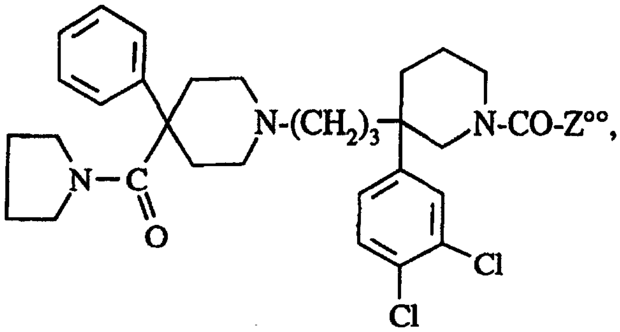

isomère (+) (I°°a)

| Exemples | Z°° | Sel, solvate ; F°C ; $[\alpha]_D^{20}$ |
|---|---|---|
| 2 | 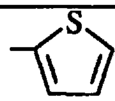 | HCl, 1 $H_2O$ ; 134-136 ; + 31° (c = 0,5 ; MeOH) |
| 3 | 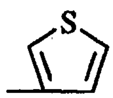 | HCl, 0,5 $H_2O$ ; 172 ; + 35,6 ° (c = 0,5 ; MeOH) |
| 4 | 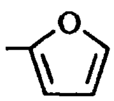 | HCl, 1,25 $H_2O$ ; 120-122 ; +47° (c = 0,5 ; MeOH) |
| 5 | 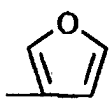 | HCl, 2 $H_2O$ ; 175 ; +32,6° (c = 0,5 ; MeOH) |

## Revendications

1. Un composé de formule :

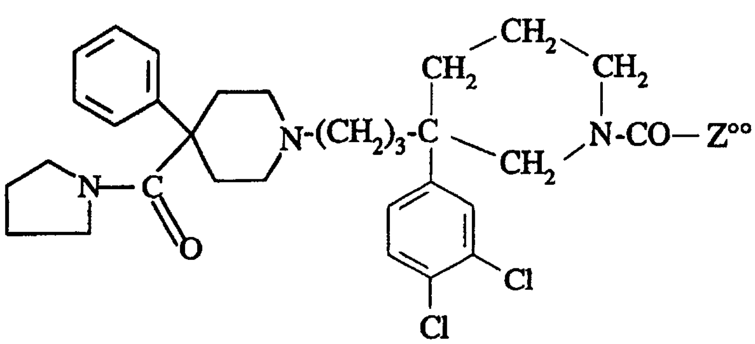

(I°°a)

dans laquelle :

- Z°° représente un 4-pyridyle, un 2-thiényle, un 3-thiényle, un 2-furyle ou un 3-furyle ;

et ses sels avec des acides minéraux ou organiques.

**2.** Un composé selon la revendication 1 choisi parmi :

la 3-(3,4-dichlorophényl)-1-isonicotinoyl-3-[3-[4-phényl-4-(pyrrolidin-1-ylcarbonyl)pipérid-1-yl]propyl]pipéridine ;
la 3-(3,4-dichlorophényl)-3-[3-[4-phényl-4-(pyrrolidin-1-ylcarbonyl)pipérid-1-yl]propyl]-1-(2-thénoyl)pipéridine ;
la 3-(3,4-dichlorophényl)-3-[3-[4-phényl-4-(pyrrolidin-1-ylcarbonyl)pipérid-1-yl]propyl]-1-(3-thénoyl)pipéridine;
la 3-(3,4-dichlorophényl)-1-(2-furoyl)-3-[3-[4-phényl-4-(pyrrolidin-1-yl-carbonyl)pipérid-1-yl]propyl]pipéridine ;
la 3-(3,4-dichlorophényl)-1-(3-furoyl)-3-[3-[4-phényl-4-(pyrrolidin-1-yl-carbonyl)pipérid-1-yl]propyl]pipéridine ;

sous forme de racémates ou de l'un de leurs énantiomères (+) ou (-),
et leurs sels avec des acides minéraux ou organiques.

**3.** Solvates des composés selon l'une des revendications 1 ou 2 et des sels de ceux-ci.

**4.** Procédé pour la préparation d'un composé de formule (I°°a) selon la revendication 1 et de ses sels, **caractérisé en ce que** :

1°) on fait réagir un composé de formule :

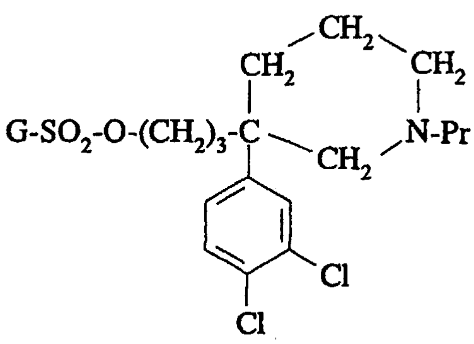

(XIX°)

dans laquelle G représente un groupe méthyle, phényle, tolyle ou trifluorométhyle et Pr représente un groupe N-protecteur, tel que le groupe trityle, *tert*-butoxycarbonyle ou benzyloxycarbonyle, avec un composé de formule :

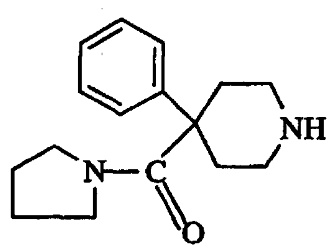

pour obtenir un composé de formule :

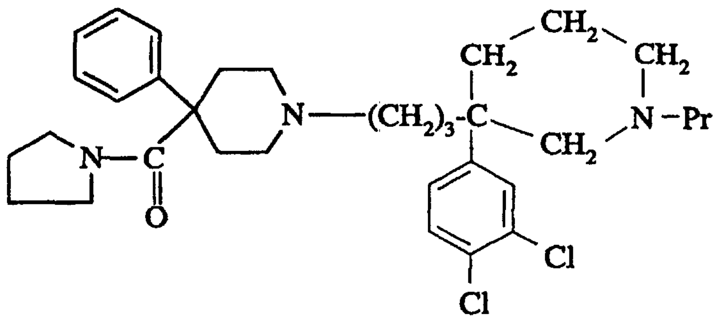

(XX°)

2•) on élimine sélectivement le groupe protecteur Pr du composé de formule (XX°), pour obtenir le composé de formule :

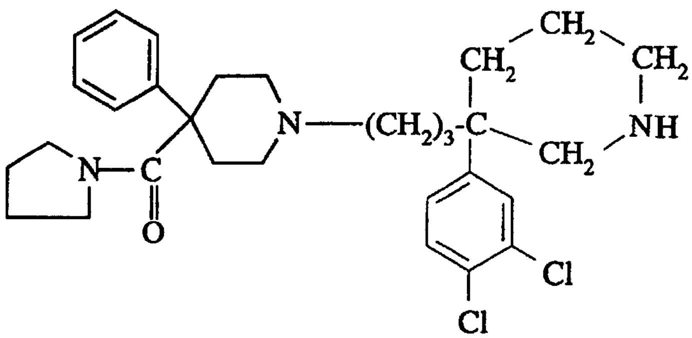

(XXI°)

3•) on traite le composé de formule (XXI°) avec un dérivé fonctionnel d'un acide de formule :

HO-CO-Z°° (IIIa)

dans laquelle Z°° est tel que défini pour un composé de formule (I°°a) dans la revendication 1 ;
4•) et on transforme éventuellement le produit (I°°a) ainsi obtenu en l'un de ses sels avec un acide minéral ou organique.

**5.** Un énantiomère d'un composé de formule :

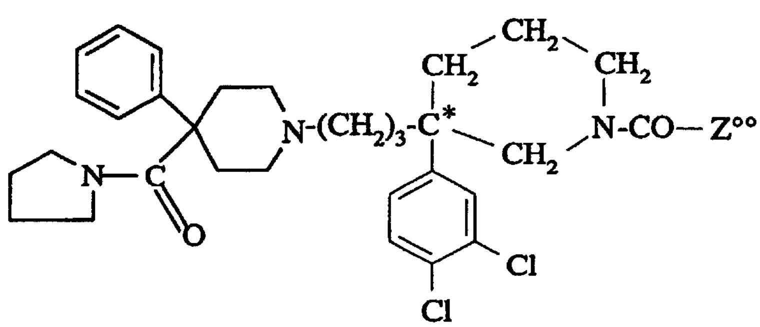

(I°°a*)

dans laquelle :

- Z°° est tel que défini dans la revendication 1 ;
  et
- "*" signifie que l'atome de carbone ainsi marqué a la configuration absolue (+) ou (-) déterminée ;

ainsi que ses sels avec des acides minéraux ou organiques et leurs solvates.

**6.** Composition pharmaceutique comprenant en tant que principe actif un composé selon l'une quelconque des revendications 1 à 3 ou 5 ou un de ses sels et solvates pharmaceutiquement acceptables.

**7.** Composition pharmaceutique selon la revendication 6, sous forme d'unité de dosage, dans laquelle le principe actif est mélangé à au moins un excipient pharmaceutique.

**8.** Composition pharmaceutique selon la revendication 7, contenant 0,5 à 1000 mg de principe actif.

**9.** Composition pharmaceutique selon la revendication 8, contenant 2,5 à 250 mg de principe actif.

**Patentansprüche**

**1.** Verbindungen der Formel:

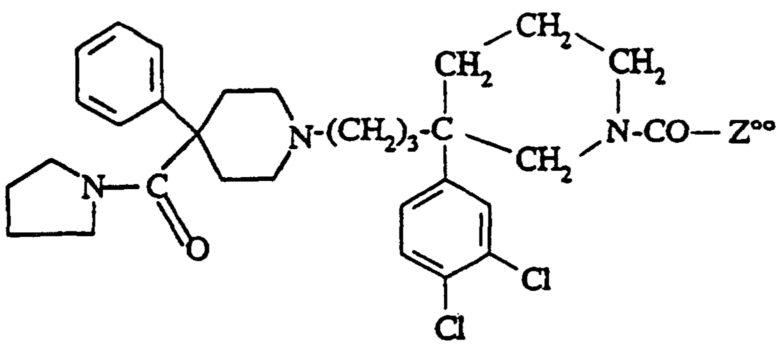

(I°°a),

worin bedeutet:

- Z°° 4-Piperidyl, 2-Thienyl, 3-Thienyl, 2-Furyl oder 3-Furyl; und

die Salze dieser Verbindungen mit anorganischen oder organischen Säuren.

**2.** Verbindungen nach Anspruch 1, die ausgewählt sind unter:

· 3-(3,4-Dichlorphenyl)-1-isonicotinyl-3-[3-[4-phenyl-4-(pyrrolidin-1-ylcarbonyl)piperid-1-yl]propyl]piperidin;
· 3-(3,4-Dichlorphenyl)-3-[3-[4-phenyl-4-(pyrrolidin-1-ylcarbonyl)piperid-1-yl]propyl]-1-(2-thenoyl)piperidin;
· 3-(3,4-Dichlorphenyl)-3-[3-[4-phenyl-4-(pyrrolidin-1-ylcarbonyl)piperid-1-yl]propyl]-1-(3-thenoyl)piperidin;
· 3-(3,4-Dichlorphenyl)-1-(2-furoyl)-3-[3-[4-phenyl-4-(pyrrolidin-1-yl-carbonyl(piperid-1-yl]propyl]piperidin;
· 3-(3,4-Dichlorphenyl)-1-(3-furoyl)-3-[3-[4-phenyl-4-(pyrrolidin-1-yl-carbonyl)piperid-1-yl]propyl]piperidin;

in Form der Racemate oder in Form eines ihrer Enantiomere (+) oder (-),
und ihre Salze mit anorganischen oder organischen Säuren.

**3.** Solvate der Verbindungen nach einem der Ansprüche 1 oder 2 und ihrer Salze.

**4.** Verfahren zur Herstellung von Verbindungen der Formel (I°°a) nach Anspruch 1 und ihrer Salze, **dadurch gekennzeichnet, dass**:

1•) eine Verbindung der Formel:

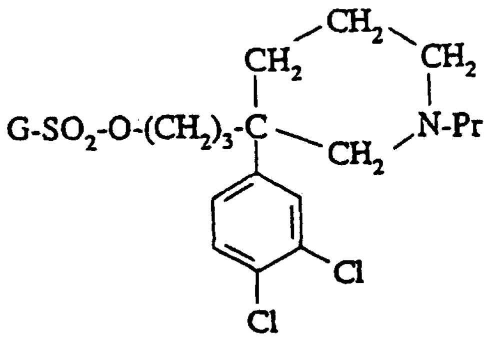

(XIX°),

worin die Gruppe G Methyl, Phenyl, Tolyl oder Trifluormethyl bedeutet und Pr eine N-Schutzgruppe ist, beispielsweise Trityl, *t*-Butoxycarbonyl oder Benzyloxycarbonyl, mit einer Verbindung der Formel:

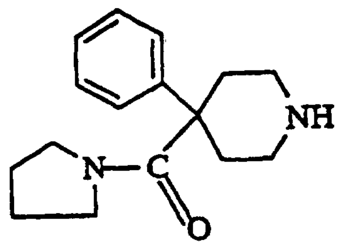

umgesetzt wird, um eine Verbindung der folgenden Formel herzustellen:

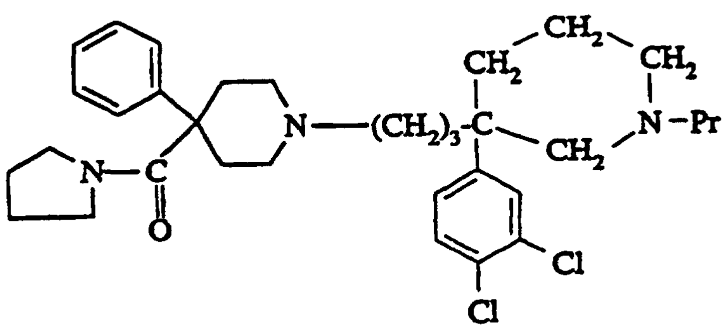

(XX°)

2•) die Schutzgruppe Pr selektiv aus der Verbindung der Formel (XX°) entfernt wird, um die Verbindung der folgenden Formel herzustellen:

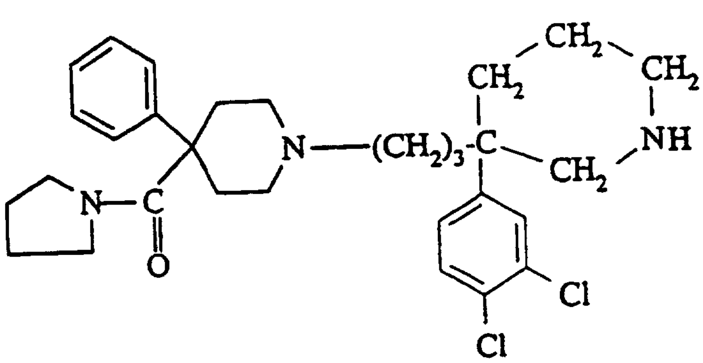

(XXI°)

3•) die Verbindung der Formel (XXI°) mit einem funktionellen Derivat einer Säure der Formel

$$HO\text{-}CO\text{-}Z^{\circ\circ} \quad \text{(IIIa)}$$

umgesetzt wird, worin die Gruppe Z°° die für die Verbindungen der Formel (I°°a) in Anspruch 1 angegebenen Bedeutungen aufweist; und

4•) das auf diese Weise erhaltene Produkt (I°°a) mit einer anorganischen oder organischen Säure in eines ihrer Salze übergeführt wird.

**5.** Enantiomere von Verbindungen der Formel:

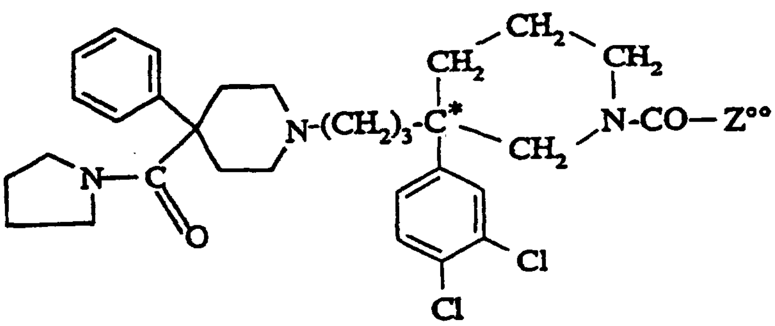

(I°°a*),

worin:

- Z°° die in Anspruch 1 angegebenen Bedeutungen hat; und
- "*" bedeutet, das das derart markierte Kohlenstoffatom die vorbestimmte Absolutkonfiguration (+) oder (-) hat;

sowie ihre Salze mit anorganischen oder organischen Säuren und ihre Solvate.

**6.** Pharmazeutische Zusammensetzung, die als Wirkstoff eine Verbindung nach einem der Ansprüche 1 bis 3 oder 5 oder eines ihrer pharmazeutisch akzeptablen Salze und Solvate enthält.

**7.** Pharmazeutische Zusammensetzung nach Anspruch 6 in Form einer Dosierungseinheit, wobei der Wirkstoff im Gemisch mit mindestens einem pharmazeutischen Excipienten vorliegt.

**8.** Pharmazeutische Zusammensetzung nach Anspruch 7, die 0,5 bis 1000 mg Wirkstoff enthält.

**9.** Pharmazeutische Zusammensetzung nach Anspruch 8, die 2,5 bis 250 mg Wirkstoff enthält.

## Claims

**1.** A compound of the formula :

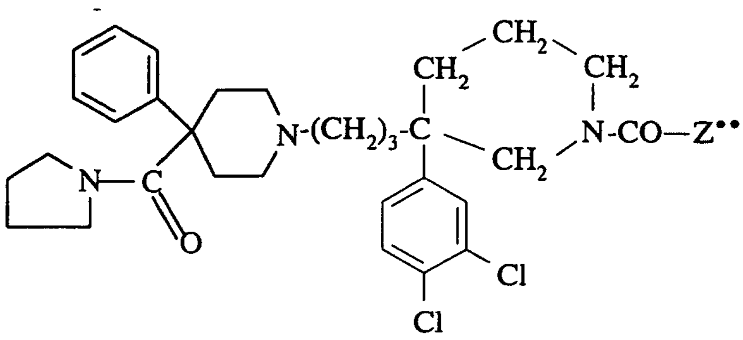

(I••a)

in which:

- Z•• represents a 4-pyridyl, 2-thienyl, 3-thienyl, 2-furyl or 3-furyl;

and its salts with mineral and organic acids.

**2.** A compound according to claim 1, selected from :

3-(3,4-dichlorophenyl)-1-isonicotinoyl-3-[3-[4-phenyl-4-(pyrrolidin-1-ylcarbonyl)piperid-1-yl]propyl]piperidine;
3-(3,4-dichlorophenyl)-3-[3-[4-phenyl-4-(pyrrolidin-1-ylcarbonyl)piperid-1-yl]propyl]-1-(2-thenoyl)piperidine;
3-(3,4-dichlorophenyl)-3-[3-[4-phenyl-4-(pyrrolidin-1-ylcarbonyl)piperid-1-yl]propyl]-1-(3-thenoyl)piperidine;
3-(3,4-dichlorophenyl)-1-(2-furoyl)-3-[3-[4-phenyl-4-(pyrrolidin-1-ylcarbonyl)piperid-1-yl]propyl]piperidine;
3-(3,4-dichlorophenyl)-1-(3-furoyl)-3-[3-[4-phenyl-4-(pyrrolidin-1-ylcarbonyl)piperid-1-yl]propyl]piperidine;

in the form of racemates or one of their (+) or (-) enantiomers,
and their salts with mineral or organic acids.

**3.** Solvates of the compounds according to claims 1 or 2 and their salts.

**4.** Method for the preparation of a compound of formula (I••a) according to claim 1 and its salts, **characterized in that**:

1•) a compound of the formula :

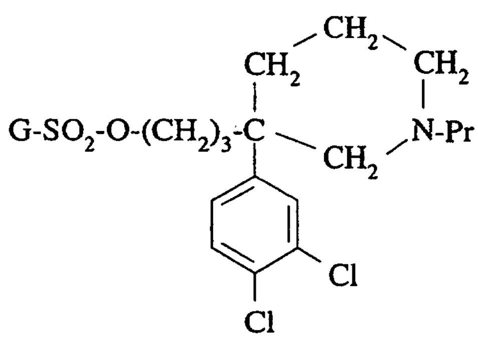

(XIX•)

in which G is a methyl, phenyl, tolyl or trifluoromethyl group and Pr is an N-protecting group such as the trityl, *tert*-butoxycarbonyl or benzyloxycarbonyl group, is reacted with a compound of the formula:

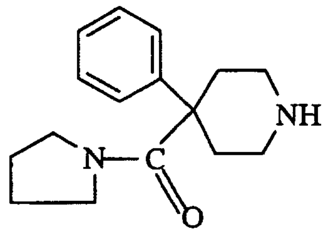

to give a compound of the formula :

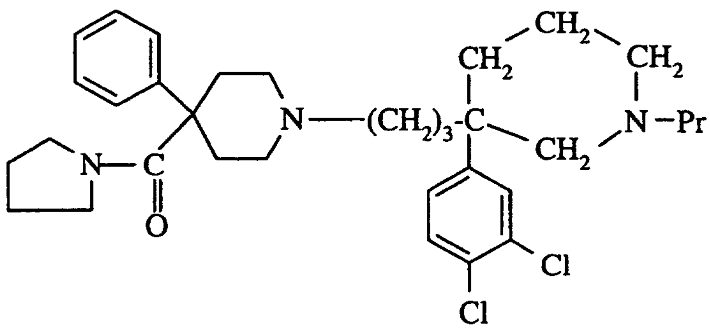

(XX°)

2•) the protecting group Pr is selectively removed from the compound of formula (XX°) to give the compound of the formula:

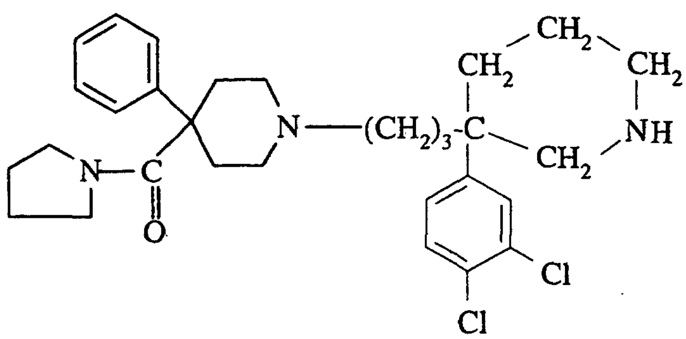

(XXI°)

3•) the compound of formula (XXI°) is treated with a functional derivative of an acid of the formula :

HO-CO-Z•• (IIIa)

in which Z•• is as defined for a compound of formula (I••a) in claim 1; and

4•) the resulting product (I••a) is optionally converted to one of its salts with a mineral or organic acid.

**5.** An enantiomer of a compound of the formula :

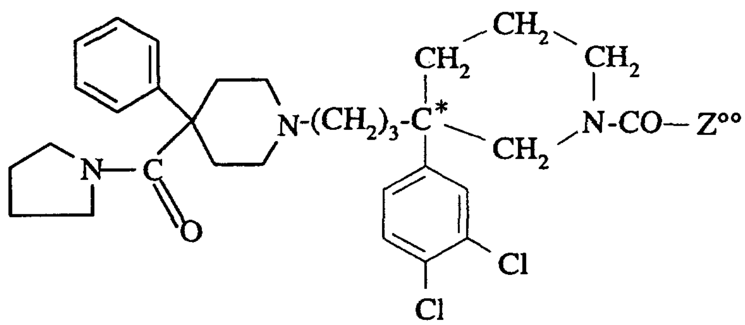 (I°°a*)

in which:

- Z°° is as defined in claim 1,
- "*" denotes that the carbon atom carrying this label has the determined (+) or (-) absolute configuration;

and its salts with mineral or organic acids, and their solvates.

**6.** Pharmaceutical composition comprising, as the active principle, a compound according to any one of claims 1 to 3 or 5 or one of its pharmaceutically acceptable salts and solvates.

**7.** Pharmaceutical composition according to claim 6 in the form of a dosage unit in which the active principle is mixed with at least one pharmaceutical excipient.

**8.** Pharmaceutical composition according to claim 7 containing 0.5 to 1000 mg of active principle.

**9.** Pharmaceutical composition according to claim 8 containing 2.5 to 250 mg of active principle.